# EUROPEAN PATENT APPLICATION

(11) **EP 1 426 367 A1**
(43) Date of publication of application: **09.06.2004**
(21) Application number: 02762804.9
(22) Date of filing: 19.08.2002
(51) Int. Cl.: C07D 307/33, C07D 409/04, C07D 307/80, A01N 43/12, A01N 43/08, A01N 43/16, A01N 43/20

(54) **LACTONE DERIVATIVE, AND PLANT GROWTH REGULATOR AND ROOTING AGENT EACH CONTAINING THE SAME AS ACTIVE INGREDIENT**

(30) Priority: 20.08.2001 JP 2001248946
(71) Applicant: SHISEIDO COMPANY, LTD., Chuo-ku, Tokyo 104-8010 (JP)
(72) Inventor: KOBAYASHI, Koji, c/o Shiseido Research Center, Yokohama-shi, Kanagawa 224-8558 (JP); YOKOYAMA, Mineyuki, c/o Shiseido Research Center, Yokohama-shi, Kanagawa 224-8558 (JP)
(74) Representative: Henkel, Feiler & Hänzel
(86) International application number: PCT/JP2002/008342
(87) International publication number: WO 2003/016297

(57) **Abstract**

The invention provides a lactone derivative of formula (I) which exhibits excellent rooting activity, and a plant growth regulator containing the derivative as an active ingredient. 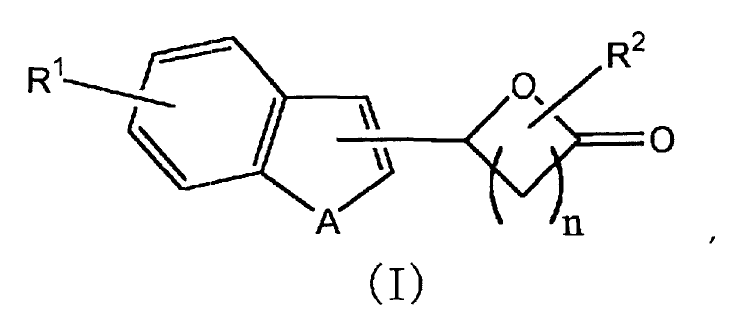 wherein R¹ and R² each individually represent a hydrogen atom, a halogen atom, an alkyl group having 1 to 6 carbon atoms, or an alkoxyl group having 1 to 6 carbon atoms; A represents a methylene group, a vinylene group, an imino group, an oxygen atom, or a sulfur atom; and n is an integer of 1 to 4;
wherein when A is an imino group, both of R¹ and R² are atoms or groups other than hydrogen, and when A is an oxygen atom, both of R¹ and R² are hydrogen atoms.

## Description

### Technical Field

The present invention relates to a lactone derivative having a specified chemical structure, and to plant-growth-related agents containing the derivative as an active ingredient.

### Background Art

Auxins such as indolebutyric acid (IBA) and naphthylacetic acid (NAA) have been used as rooting agents for cuttings; and yet, many plants have difficulty in developing roots. Such plants are primarily found among woody plants, and examples thereof include *myrica rubra*, walnut trees, *Betulaceace* (*Carpinus japonica, Alnus firma*, etc.), beeches (chestnut trees, oaks, etc.), elms (such as hackberries), magnolias (*Magnolia obovata, Liriodendron tulipifera, Illicium religiosum*, etc.), pine trees (fir trees, *Tsuga sieboldii, Larix kaempferi, Pinus thunbergii,* etc.), and sweet osmanthus. At present, lumber for building purposes is produced only from plantings of plants that can be grown from cuttings. Thus, discovery of a new rooting agent would certainly enable new varieties of lumber to be developed.

Generally, auxins are believed to contribute to the mechanism of rooting. From the finding that the effect of IBA, a type of auxin, on a plant is significantly promoted when the plant is in an oxidation state (i.e., a state where active oxygen is generated), in order for an auxin to exhibit its effect of inducing rooting, generation of active oxygen might be necessary. Also, according to one theory related to the action mechanism of auxins, auxins perform a certain function in the apoplast (extracellular fluid) before they are taken into cells (Sakurai, N. (1998), Dynamic Function and Regulation of Apoplast in the Plant Body, J. Plant Res. 111: 133-148). Having been interested in these theories, we hypothesized that under conditions where active oxygen is generated, IBA provided from the outside may undergo some metabolism before it is taken in the cells. We previously investigated the possible presence of IBA metabolites produced in an initial stage in the apoplast, and discovered a new auxin metabolite (hereinafter abbreviated as Fb) which itself exhibits the effect of inducing rooting (Japanese Patent Application Laid-Open (*kokai*) No. 10-77268).

Under the co-presence of IBA, the novel rooting substance (Fb) was found to potentiate its activity (increase the number of developed roots) and increase the weight of developed roots and weight of seedlings. Furthermore, it has been found that Fb, unlike IBA, is effective when sprayed onto above-ground portions of a plant rather than deposited on root portions, and therefore, in practice, is more conveniently handled than is IBA.

However, depending on the conditions under which Fb is used, the effect of Fb, if used alone, may be weaker than that of IBA, an existing rooting agent. Thus, objects of the present invention include provision of a compound exhibiting a rooting activity superior to that of Fb, and provision of a plant growth regulator exhibiting rooting activity and containing the compound as an active ingredient.

### Disclosure of the Invention

In order to solve the above-mentioned problems, the present inventors have performed extensive research in an attempt to develop new rooting agent based on the structure of Fb, and have found that lactone derivatives of a certain new class exhibit a strong rooting activity, thus leading to completion of the present invention.

Accordingly, the present invention provides a lactone derivative represented by the following formula (I) (hereinafter may be referred to as the "present lactone derivative"): 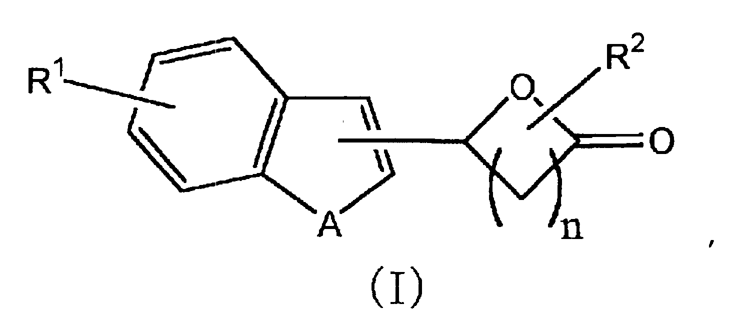 wherein R¹ and R² each individually represent a hydrogen atom, a halogen atom, an alkyl group having 1 to 6 carbon atoms, or an alkoxyl group having 1 to 6 carbon atoms; A represents a methylene group, a vinylene group, an imino group, an oxygen atom, or a sulfur atom; and n is an integer of 1 to 4;
wherein when A is an imino group, both of R¹ and R² are atoms or groups other than hydrogen, and when A is an oxygen atom, both of R¹ and R² are hydrogen atoms.

The present invention also provides a plant growth regulator containing, as an active ingredient, the present lactone derivative (hereinafter the regulator may be referred to as the "present growth regulator"). Preferably, the present growth regulator is embodied as a rooting agent endowed with rooting induction activity (hereinafter may be referred to as the "present rooting agent").

Substances which can be used as the active ingredient of the present growth regulator or the present rooting agent are not necessarily limited to the present lactone derivatives. Instead, they may be lactone derivatives of formula (I) in which A is an oxygen atom and at least one of R¹ and R² is an atom, or a group, other than a hydrogen atom. Thus, for the sake of convenience, those lactone derivatives which, in a strict sense of the term, fall outside the definition of the present lactone derivative but can still serve as the above-mentioned active ingredient are also referred to as the "present lactone derivative." Therefore, the descriptions including production methods which follow herein may cover such a broad sense of the term.

### Best Mode for Carrying Out the Invention

Modes for carrying out the invention will next be described.

In the present lactone derivatives represented by formula (I), each of R¹ and R² is a hydrogen atom, a halogen atom, an alkyl group having 1 to 6 carbon atoms, or an alkoxyl group having 1 to 6 carbon atoms. Preferably, R¹ is a hydrogen atom or a halogen atom, and R² is a hydrogen atom.

In formula (I), the halogen atom is a fluorine atom, a chlorine atom, a bromine atom, or an iodine atom. Of these, a fluorine atom and a chlorine atom are particularly preferred.

The alkyl group having 1 to 6 carbon atoms (hereinafter may be referred to as the C₁₋₆ alkyl group) may be linear or branched, and examples of the C₁₋₆ alkyl group include methyl, ethyl, n-propyl, isopropyl, n-butyl, isobutyl, sec-butyl, tert-butyl, n-pentyl, isoamyl, and n-hexyl. Other examples of the C₁₋₆ alkyl group include cyclic alkyl groups, and examples of the cyclic alkyl groups include cyclopropyl, cyclobutyl, cyclopentyl, and cyclohexyl. A preferred example of the C₁₋₆ alkyl group is methyl.

Examples of the alkoxyl group having 1 to 6 atoms (hereinafter may be referred to as the C₁₋₆ alkoxyl group) include hydroxyl groups whose hydrogen atom has been substituted by the above-mentioned C₁₋₆ alkyl group. Specific examples of the C₁₋₆ alkoxyl group include methoxy, ethoxy, isopropoxy, and isobutyloxy. A preferred example of the C₁₋₆ alkoxyl group is methoxy.

"A" of the present lactone derivatives is methylene (-CH₂-), vinylene (-CH=CH-), imino (-NH-), an oxygen atom, or a sulfur atom, preferably methylene, vinylene, an oxygen atom, or a sulfur atom, particularly preferably an oxygen atom or a sulfur atom. When "A" is an oxygen atom, both of R¹ and R² are preferably hydrogen atoms.

"n" of the present lactone derivatives is 1 to 4, preferably 2.

At least one asymmetric carbon atom may be present in the present lactone derivative. The present lactone derivative encompasses isomers thereof based on the asymmetric carbon atom; i.e., optical isomers thereof, conformational isomers thereof, and mixtures of any of the optical isomers and conformational isomers.

The present lactone derivative may be produced through common methods by use of known materials as starting materials.

A typical process for producing the present lactone derivative is shown in Production Process (α), and other related production processes will also be described. It should be noted that the process for producing the present lactone derivatives may be suitably determined in consideration of the type of the lactone derivative to be produced, the starting materials used in production, the production scale of the lactone derivative, or other factors, and the present invention should not be construed as being limited only to the specific processes described herein. Unless otherwise specified, in the following description of production processes, R¹, R², A, and n have the same meanings as defined in formula (I).

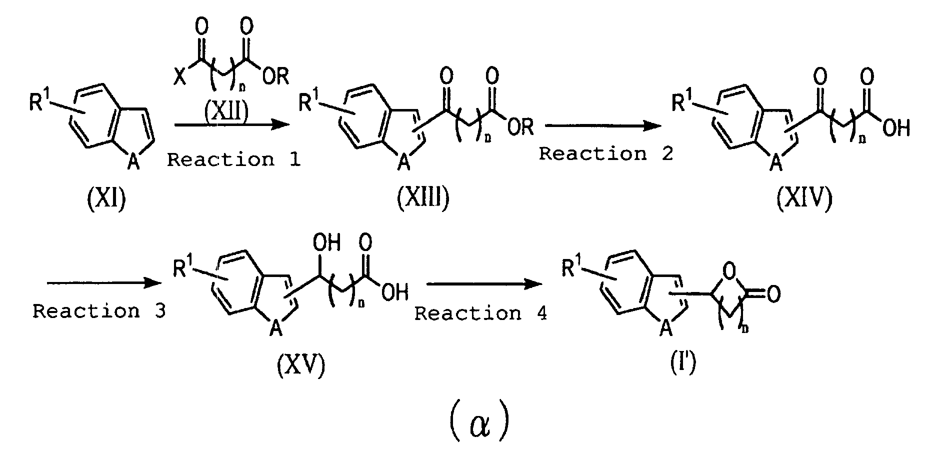

In reaction (1), compound (XI) is reacted with acid halide (XII) to thereby yield keto-ester (XIII). Reaction (1), which is acylation of an aromatic ring named as Friedel-Crafts reaction, may be performed through the method described in "Friedel-Crafts and Related Reactions", I-III (authored by G. A. Olah, Interscience Publisher Inc., New York, 1963-1965). Specifically, an aromatic compound (XI) is reacted with an acid halide (XII) or a corresponding acid anhydride in the presence of a catalyst (such as aluminum chloride, aluminum bromide, tin tetrachloride, or boron trifluoride) in a solvent (such as carbon disulfide, nitrobenzene, benzene, chloroform, methylene chloride, or carbon tetrachloride) at a temperature from -20°C to the reflux temperature, to thereby yield a target compound. X of the acid halide (XII) represents a halogen atom (a fluorine atom, a chlorine atom, a bromine atom, or an iodine atom), among which acid chloride (X=chlorine) is most widely employed. R represents a group for protecting the carboxylic acid moiety, and no particular limitation is imposed on the species of R, so long as the protecting group does not raise any problem in reactions for producing the present lactone derivative. For example, R may be a protecting group described in "Protecting Groups in Organic Synthesis" (authored by T. W. Greene, published by John Wiley & Sons).

When the acid halide (XII) is replaced by a corresponding dicarboxylic acid anhydride (such as malonic anhydride, succinic anhydride, or glutaric anhydride) and reaction is allowed to proceed with a compound (XI) under appropriate conditions, a keto-acid (XIV) can be obtained directly.

Also, when the compound (XI) is transformed to a corresponding aromatic metal compound (such as a Grignard derivative or a corresponding lithium compound) and then allowed to react with a dicarboxylic acid derivative (such as an acid chloride or an acid anhydride), a keto-ester (XIII) or a keto-acid (XIV) can also be obtained.

Such a Grignard derivative can be obtained by, among other methods, reacting a halogenated alkyl (such as ethyl iodide or ethyl bromide) with metallic magnesium to thereby yield a Grignard reagent, and reacting the reagent with a compound (XI). The Grignard derivative is reacted with a dicarboxylic acid derivative (e.g., an acid halide (XII) or an acid anhydride such as malonic anhydride, succinic anhydride, or glutaric anhydride), to thereby yield a keto-ester (XIII) or a keto-acid (XIV).

In the above reaction, when "A" is imino (-NH-), and neither a Grignard reaction nor a lithium reaction described above is employed in the reaction of acid halide (XII) with compound (XI), the imino group is preferably protected in advance in accordance with needs. Examples of the protecting group used in this case include triisopropylsilyl, tert-butyldiphenylsilyl, tert-butyldimethylsilyl, and [2-(trimethylsilyl)ethoxy]methyl.

Reaction 2 is a deprotection reaction―from keto-ester (XIII) to keto-acid (XIV)―required when the above reaction proceeds via keto-ester (XIII) (an intermediate product), and may be performed through the method described in the above-mentioned "Protecting Groups in Organic Synthesis" (authored by T. W. Greene, published by John Wiley & Sons). For example, when R is alkyl such as methyl or ethyl, reaction is performed in the presence of a base (such as sodium hydroxide or lithium hydroxide) in a solvent (such as water, dioxane, methanol, or ethanol) under cooling with ice or at a temperature up to the reflux temperature, whereby the deprotection can be accomplished.

Reaction 3 is a reduction reaction in which keto-acid (XIV) is reduced to hydroxy acid (XV). In the reaction, a reducing agent that reduces carbonyl preferentially to carboxylic acid moiety is employed. For example, reaction is performed by use of a reducing agent (such as sodium borohydride (NaBH₄), lithium borohydride (LiBH₄), or sodium cyanoborohydride (NaBH₃CN)) in a solvent (such as water, alcohol (methanol, ethanol), ether (tetrahydrofuran, 2-methoxyethyl ether), or hexamethylphosphoramide) under cooling with ice or at a temperature up to the reflux temperature, whereby the carbonyl group can be reduced.

When carbonyl is stereoselectively reduced, there may be employed, for example, a method using diisobutylaluminum hydride (DIBAL-H) in the presence of zinc chloride (reported in "Erythro-selectivity" R. Frenette et al., J. Org. Chem., 1987, 52, 304-307). Through such a reaction, lactone derivatives that are optically active can be produced.

In reaction 4, the hydroxy acid (XV) is dehydrated, forming a lactone ring, to thereby yield the lactone derivative (I') of the present invention. The reaction can be performed, for example, in a solvent (such as benzene or toluene) in the presence of, among others, p-toluenesulfonic acid or sulfuric acid under cooling with ice or at a temperature up to the reflux temperature.

The target compound (I') may be produced directly from compound (XIII), instead of via compound (XIV) or (XV), through a method which employs, for example, tetrabutylammonium borohydride (D. J. Raber et al., J. Org. Chem., 1976, 41, 690-696).

The above reaction scheme represents the case in which R² is a hydrogen atom. When, instead of compound (XII), compound (XII') is employed―wherein the compound (XII') has a corresponding alkylene chain that has been substituted with a substituent R² other than hydrogen―, the present lactone derivative of interest can be produced.

The starting materials in the above production process; i.e., compounds (XI), (XII), and (XII'), may be commercially available compounds or may be prepared through known reactions by use of known starting materials.

The present lactone derivatives exhibit excellent rooting activity, and thus are useful as rooting agents or plant growth regulators.

A plant growth regulator is an agent which has an effect of promoting or inhibiting growth of the whole plant or any part of plant, and promotes or inhibits, for example, growth of the plant as a whole, formation of flower buds, stimulation of rooting, or dormancy. A rooting agent is an agent which induces rooting of plants, and can be used to facilitate, for example, growing a plant from a cutting such as a stem cutting. A rooting agent can also be used to facilitate rooting of woody plants planted in areas where the plants are to be grown.

When the present lactone derivative is employed as an active ingredient of a plant growth regulator (the present growth regulator) or a rooting agent (the present rooting agent), the present lactone derivative per se may be used as the present growth regulator or rooting agent, or the present lactone derivative may be processed into a composition having a target form which may be applied to plants, such as liquid, solid, powder, or emulsion. Regardless of the form the present growth regulator or rooting agent may take, the lactone derivative should preferably remain stable in the regulator or agent.

So long as the stability of the present lactone derivative is ensured, generally known carrier components, additives for formulation, etc. may be added appropriately in accordance with the target form. Examples of the carrier components which may be employed include solid carriers such as inorganic substances (e.g., talc, clay, vermiculite, diatomaceous earth, kaolin, calcium carbonate, calcium hydroxide, terra alba, and silica gel), as well as wheat powder and starch; and liquid carriers such as water, aromatic hydrocarbons (e.g., xylene), alcohols (e.g., ethanol, ethylene glycol), ketones (e.g., acetone), ethers (e.g., dioxane, tetrahydrofuran), dimethylformamide, dimethylsulfoxide, and acetonitrile. In addition, buffers for maintaining a constant pH may be employed.

Examples of the additives for formulation include anionic surfactants such as alkyl sulfate, alkyl sulfonate, alkylaryl sulfonate, and dialkyl sulfosuccinate; cationic surfactants such as salts of higher fatty acid amines; nonionic surfactants such as polyoxyethylene glycol alkyl ethers, polyoxyethylene glycol acyl esters, polyoxyethylene glycol polyhydric alcohol acyl esters, and cellulose derivatives; thickners such as gelatin, casein, and acacia; bulking agents; and binders. These additives may be appropriately incorporated according to needs.

In addition, known plant growth regulators such as benzoic acid, nicotinic acid, nicotinamide, and pipecolic acid may be added to the present growth regulator or rooting agent, so long as they do not inhibit the effect of the present lactone derivative.

The present growth regulator or rooting agent is applied to various plants in a manner appropriate to the form thereof. For example, the regulator or agent in the form of powder or liquid is applied to cut surfaces of the vegetative organs of the plant to induce rooting, and the regulator or agent in the form of liquid may also be sprayed to the above-ground portion of the plant. The sprayability of the present growth regulator or rooting agent is an advantageous feature attributed to the present lactone derivative as contrasted to conventional rooting agents such as Oxyberon (component: IBA), which is not suitable to be sprayed. The present growth regulator or rooting agent is used singly or in combination with a conventional rooting agent (e.g., Oxyberon). In such a case of combined use (e.g., together with Oxyberon), good results can often be obtained through application of the conventional rooting agent to the base portion of the cutting and spraying of the present growth regulator or rooting agent in liquid form onto the above-ground portion of the plant. In use of the present growth regulator or rooting agent, the concentration of the present lactone derivative contained therein is typically 1 to 1,000 ppm, preferably 10 to 500 ppm. The above manner of use may also be applicable to the novel auxin metabolite (Fb) described in Japanese Patent Application Laid-Open (*kokai*) No. 10-77268.

No particular limitation is imposed on the species of the plants to which the present growth regulator or rooting agent is applied, and the regulator or agent is effective for both dicotyledon plants and monocotyledon plants.

### Examples

The present invention will next be described in more detail by way of examples.

### Rooting Activity Test

### (a) Plant and Culture conditions

In order to evaluate rooting activity, adventitious roots of *Bupleurum falcatum* L. were employed. The adventitious roots were obtained by growing the roots of aseptic seedlings by use of IBA. The developed roots were cultured at 23°C in a B5 liquid medium containing 5 mg/L IBA (for 3 weeks) and in the corresponding B5 liquid medium with no IBA (for 6 weeks) in an alternate manner. The inoculation amount of the roots was 5 g/L, the incubation temperature was 23°C, and subculture was performed in the dark under shaking conditions of 100 rpm.

### (b) Rooting test using Bupleurum falcatum L. (1)

In the rooting test, adventitious roots cultured in IBA-free medium were employed. B5 medium (30-mL) was placed in a 100 mL flask, followed by planting of the roots of *Bupleurum falcatum* L. (0.2 g) under aseptic conditions. The present lactone derivative, prepared in 10,000 ppm, was added to the flask so as to attain the concentration of 4 ppm or 8 ppm. A sample containing a 4 ppm (final concentration) IBA solution and a sample containing a 8 ppm (final concentration) IBA solution were employed as positive controls, and a corresponding IBA-free sample was employed as a control. Respective samples were cultured under shaking at 100 rpm at 23°C in the dark, and on day 24, the extent of rooting was checked. The roots were freeze-dried and the weight thereof was measured.

### (c) Results (1)

The results obtained from tests using compound 1 (prepared through below-described Production Example 1) and compound 2 (prepared through below-described Production Example 2), which are the present lactone derivatives, as well as the results obtained from tests using IBA alone are shown in Table 1.

As is apparent from Table 1, all the tested lactone derivatives of the present invention exhibited rooting activity, and in particular, compound 2 exhibited rooting activity stronger than IBA, which was used as the positive control, in a dose-dependent manner.

**Table 1**

| | Degree of Rooting | Weight of Weight of Freeze-dried Roots (g) |
|---|---|---|
| IBA (4 ppm) | ++ | 0.31 |
| IBA (8 ppm) | +++ | 0.37 |
| Compound 1 (4 ppm) | + | 0.37 |
| Compound 1 (8 ppm) | ± | 0.35 |
| Compound 2 (4 ppm) | ++ | 0.36 |
| Compound 2 (8 ppm) | ++++ | 0.41 |

### (d) Rooting test using Bupleurum falcatum L. (2)

The general procedure of the above-described rooting test (1) was followed, except that there were employed, as positive controls, samples containing the novel auxin metabolite (Fb) disclosed in Japanese Patent Application Laid-Open (*kokai*) No. 10-77268, at 4 ppm and 8 ppm (both final concentration).

### (e) Results (2)

Table 2 shows the results obtained from the test using compound 2, which is the present lactone derivative, and from the test using Fb. The number of lateral roots was determined by averaging 3 measurements of the number of the lateral roots appearing per unit length (2 cm) of the planted root.

As is apparent from Table 2, compound 2 exhibited rooting activity stronger than Fb, which was used as the positive control, in a dose-dependent manner.

**Table 2**

| | Number of Lateral Roots | Weight of Freeze-dried Roots (g) |
|---|---|---|
| Fb (4 ppm) | 25.0 | 0.33 |
| Fb (8 ppm) | 27.3 | 0.34 |
| Compound 2 (4 ppm) | 30.0 | 0.31 |
| Compound 2 (8 ppm) | 43.3 | 0.45 |

### Production Example 1

### 5-(1-Benzofuran-2-yl)dihydro-2(3H)-furanone (compound 1)

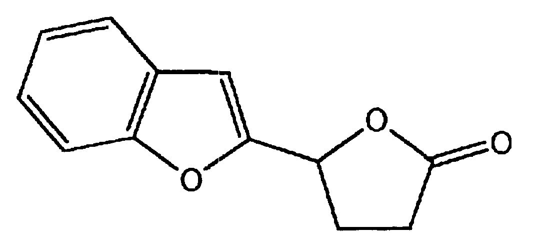

2,3-Benzofuran (3.0 g, 25.4 mmol) was dissolved in methylene chloride. To the resultant solution, ethyl 4-chloro-4-oxobutyrate (4.0 mL, 27.9 mmol) and BF3·Et20 (3.9 mL, 30.5 mmol) were added, and the mixture was stirred for 10 minutes at 0°C. Tin tetrachloride (3.6 mL, 27.9 mmol) was added dropwise thereto, the mixture was stirred for 30 minutes at 0°C, and then stirred for 2 hours at room temperature. Under cooling with ice, 3N hydrochloric acid was slowly added to the resultant mixture. Subsequently, the mixture was extracted with chloroform. After the extract was washed with water, the washed matter was dried over sodium sulfate anhydrate, filtered, and concentrated under reduced pressure. The thus-obtained crude product was subjected to silica gel column chromatography (n-hexane:ethyl acetate = 5:1), to thereby yield ethyl 4-(1-benzofuran-2-yl)-4-oxobutyrate (1.88 g, 30%).

The above-obtained ethyl 4-(1-benzofuran-2-yl)-4-oxobutyrate (1.85 g, 7.51 mmol) was dissolved in a solution of dioxane-water (1:1). To the resultant mixture, 1M lithium hydroxide (15 mL, 15 mmol) was added. After the mixture was stirred for one hour at room temperature, the resultant mixture was washed with ether. Subsequently, an aqueous citric acid solution was added to the mixture, to thereby regulate the pH of the mixture to 3-4. The mixture was extracted with ethyl acetate, and the extract was washed with saturated brine, dried over sodium sulfate anhydrate, and then concentrated under reduced pressure, to thereby yield 4-(1-benzofuran-2-yl)-4-oxobutyric acid (0.80 g, 49%).

The 4-(1-benzofuran-2-yl)-4-oxobutyric acid (0.80 g, 3.63 mmol) was dissolved in ethanol. To the solution, sodium borohydride (0.40 g, 10.9 mmol) was added at 0°C, and the mixture was stirred for 15 minutes. 1N Hydrochloric acid was added to the resultant mixture, and the salt that precipitated was separated from the mixture through filtration. An aqueous citric acid solution was added to the filtrate. The resultant mixture was extracted with ethyl acetate. The extract was washed with saturated brine, dried over sodium sulfate anhydrate, and then concentrated under reduced pressure. The residue was dissolved in toluene, p-toluenesulfonic acid (0.28 g, 1.45 mmol) was added thereto. Subsequently, the mixture was stirred for 30 minutes at room temperature. To the resultant mixture, ether was added, and the mixture was washed with saturated brine. Subsequently, the mixture was dried over sodium sulfate anhydrate, and concentrated under reduced pressure. The thus-obtained crude product was subjected to silica gel column chromatography (n-hexane:ethyl acetate = 3:1), to thereby yield the title compound as white solid (0.65 g, 89%, total yield: 13.0%)

The ¹H-NMR data of the title compound are shown below.
¹H NMR (400 MHz, CDCl3, δ): 2.58 (m, 2'-H2), 2.58, 2.83 (m, 3'-H2), 5.62 (dd, J=6.4, 6.8, 1'-H), 6.76 (s, 3-H), 7.24 (dd, J=7.2, 7.2, 5-H), 7.31 (dd, J=7.2, 8.0, 6-H), 7.46 (d, J=8.0, 7-H), 7.56 (d, J=7.2, 4-H).

### Production Example 2

### 5-(1-Benzothiophen-3-yl)dihydro-2(3H)-furanone (compound 2)

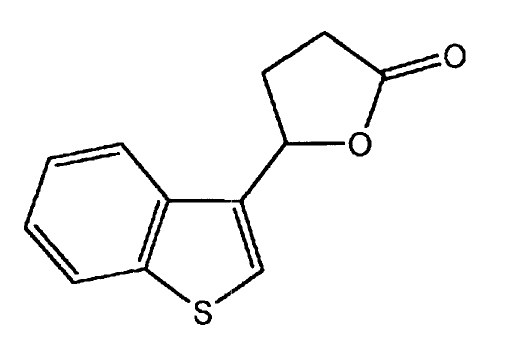

Benzo[b]thiophene (2.00 g, 14.9 mmol) was dissolved in methylene chloride. To the resultant solution, ethyl 4-chloro-4-oxobutyrate (2.34 mL, 16.4 mmol) was added, and the mixture was stirred for 10 minutes at 0°C. Tin tetrachloride (2.09 mL, 17.9 mmol) was added dropwise thereto. The mixture was stirred for 30 minutes at 0°C, and then stirred for 2 hours at room temperature. Under cooling with ice, 3N hydrochloric acid was slowly added to the resultant mixture. Subsequently, the mixture was extracted with chloroform. The extract was washed with water, followed by drying over sodium sulfate anhydrate, filtering, and concentrating under reduced pressure. The thus-obtained crude product was subjected to silica gel column chromatography (n-hexane:ethyl acetate = 5:1), to thereby yield ethyl 4-(1-benzothiophen-3-yl)-4-oxobutyrate (3.13 g, 80%).

The above-obtained ethyl 4-(1-benzothiophen-3-yl)-4-oxobutyrate (3.10 g, 11.8 mmol) was dissolved in a solution of dioxane-water (1:1). To the resultant mixture, 1M lithium hydroxide (23.6 mL, 23.6 mmol) was added, and the mixture was stirred for 30 minutes at room temperature. After washing the mixture with ether, the pH of the mixture was regulated to 3-4 through addition of an aqueous citric acid solution thereto. The resultant mixture was extracted with ethyl acetate. The extract was washed with water, followed by drying over sodium sulfate anhydrate, filtering, and concentrating under reduced pressure, to thereby yield 4-(1-benzothiophen-3-yl)-4-oxobutyric acid (2.26 g, 81%).

The above-obtained 4-(1-benzothiophen-3-yl)-4-oxobutyric acid (1.00 g, 4.2 mmol) was dissolved in ethanol. To the solution, sodium borohydride (0.48 g, 12.7 mmol) was added at 0°C, and the mixture was stirred for 15 minutes. 1N HCl was added to the resultant mixture, and the salt that precipitated was separated from the mixture through filtration. Subsequently, an aqueous citric acid solution was added to the filtrate. The resultant mixture was extracted with ethyl acetate. The extract was washed with water, followed by drying over sodium sulfate anhydrate, filtering, and then concentrating under reduced pressure. The residue was dissolved in toluene. p-Toluenesulfonic acid (0.32 g, 1.7 mmol) was added to the solution, and the mixture was stirred for 30 minutes at room temperature. To the resultant mixture, ether was added. The mixture was washed with saturated brine, followed by drying over sodium sulfate anhydrate, and then concentrating under reduced pressure. The thus-obtained crude product was subjected to silica gel column chromatography (n-hexane:ethyl acetate = 2:1), to thereby yield the title compound (0.86 g, 92%, total yield: 60%) as white needles.

The ¹H-NMR data of the title compound are shown below.
¹H NMR (400 MHz, CDCl3, δ): 2.73 (m, 2'-H2), 2.42, 2.74 (m, 3'-H2), 5.87 (dd, J=6.8, 7.6, 1'-H), 7.25 (s, 2-H), 7.40 (m, 5-H, 6-H), 7.74 (d, J=8.4, 4-H), 7.88 (d, J=8.4, 7-H).

The present lactone derivatives other than those that can be prepared through the production processes of the above-described can be prepared easily by modifying the conditions (material employed, reaction conditions, and the like) in accordance with the target product and by appropriately employing synthetic means known *per se*. Some of such lactone derivatives of the present invention are shown below by way of referential examples.

### Referential Example 1

### 6-(1-Benzothiophen-3-yl)tetrahydro-2H-pyran-2-one

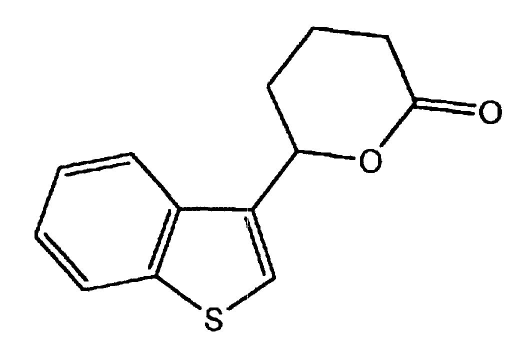

The title compound can be produced through a sequential process similar to that of Production Example 2, by use of ethyl glutaryl chloride instead of ethyl 4-chloro-4-oxobutyrate.

### Referential Example 2

### 6-(1-Benzothiophen-3-yl)-3,3,4,4,5,5-hexafluorotetrahydro-2H-pyran-2-one

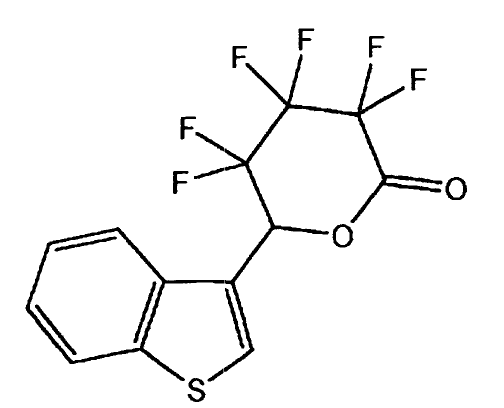

The title compound can be produced through a sequential process similar to that of Production Example 2, by use of 4-carbethoxyhexafluorobutyryl chloride instead of ethyl 4-chloro-4-oxobutyrate.

### Referential Example 3

### 4-(1-Benzothiophen-3-yl)-2-oxetanone

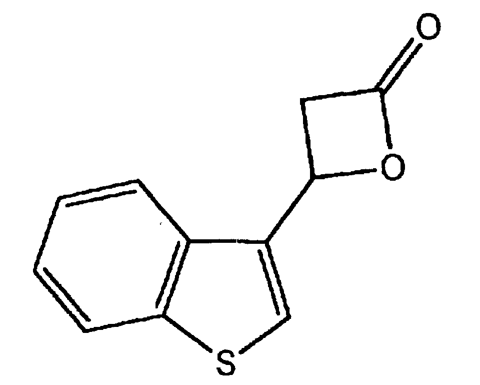

The title compound can be produced through a sequential process similar to that of Production Example 2, by use of ethyl malonyl chloride instead of ethyl 4-chloro-4-oxobutyrate.

### Referential Example 4

### 5-(4,6-Dichloro-1-benzofuran-2-yl)dihydro-2(3H)-furanone

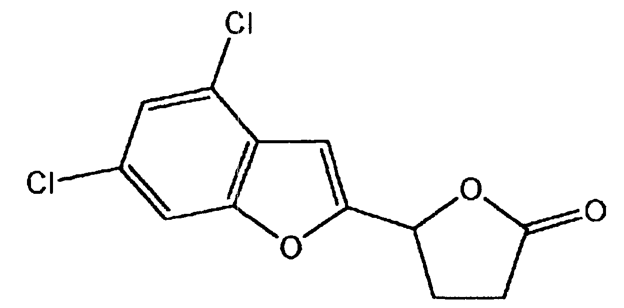

The title compound can be produced through a sequential process similar to that of Production Example 1, by use of 4,6-dichloro-1-benzofuran instead of 2,3-benzofuran.

### Referential Example 5

### 5-(5-Methoxy-1-benzofuran-2-yl)dihydro-2(3H)-furanone

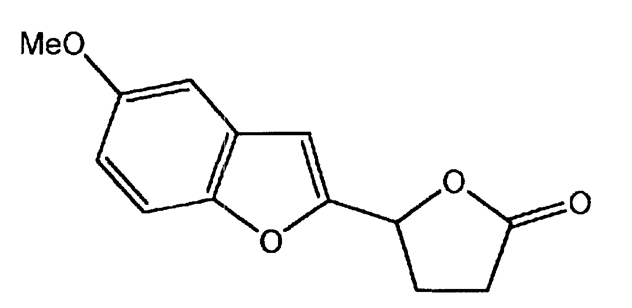

The title compound can be produced through a sequential process similar to that of Production Example 1, by use of 5-methoxy-1-benzofuran instead of 2,3-benzofuran.

### Referential Example 6

### 5-(4,6-Dimethyl-1-benzothiophen-3-yl)dihydro-2(3H)-furanone

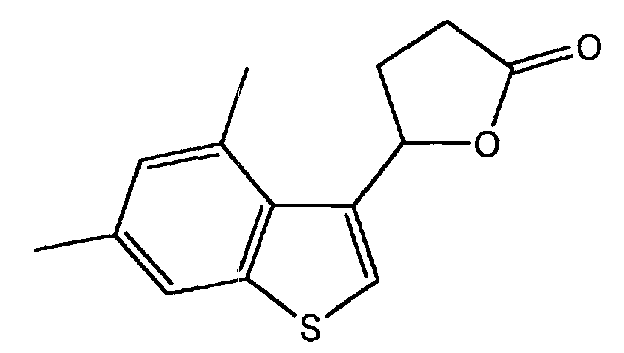

The title compound can be produced through a sequential process similar to that of Prodution Example 2, by use of 4,6-dimethyl-1-benzothiophene instead of benzo[b]thiophene.

### Referential Example 7

### 5-(1-Naphthyl)dihydro-2(3H)-furanone

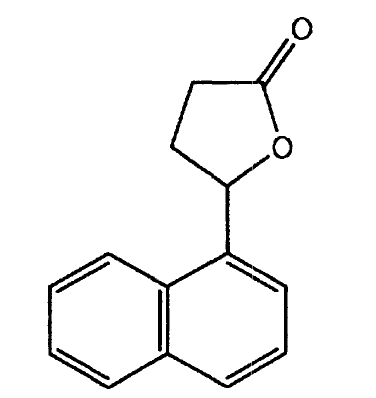

The title compound can be produced through a sequential process similar to that of Production Example 2, by use of naphthalene and aluminum chloride instead of benzo[b]thiophene and tin tetrachloride, respectively.

### Industrial Applicability

The present invention provides a lactone derivative which has rooting activity, and also provides a plant growth regulator and a rooting agent containing the derivative as an active ingredient.

## Claims

1. A lactone derivative represented by the following formula (I): 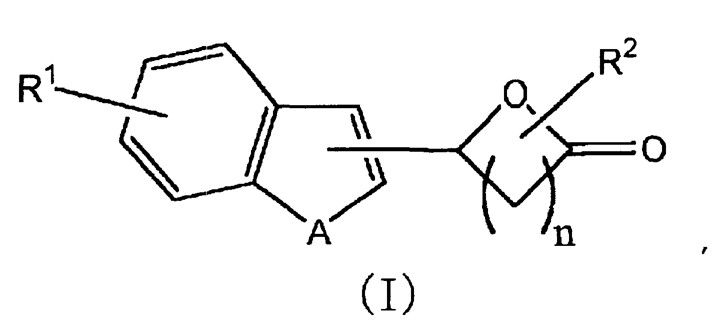 wherein R¹ and R² each individually represent a hydrogen atom, a halogen atom, an alkyl group having 1 to 6 carbon atoms, or an alkoxyl group having 1 to 6 carbon atoms; A represents a methylene group, a vinylene group, an imino group, an oxygen atom, or a sulfur atom; and n is an integer of 1 to 4;
wherein when A is an imino group, both of R¹ and R² are atoms or groups other than hydrogen, and when A is an oxygen atom, both of R¹ and R² are hydrogen atoms.

2. The lactone derivative as recited in claim 1, wherein A in the formula (I) denotes a methylene group, a vinylene group, an oxygen atom, or a sulfur atom.

3. The lactone derivative as recited in claim 1 or 2, wherein n is 2.

4. The lactone derivative as recited in any of claims 1 to 3, wherein A is an oxygen atom or a sulfur atom.

5. A plant growth regulator containing, as an active ingredient, a lactone derivative as recited in any of claims 1 to 4.

6. The plant growth regulator as recited in claim 5, which is a rooting agent.
